# EUROPÄISCHE PATENTANMELDUNG

(11) **EP 2 153 807 A1**
(43) Veröffentlichungstag der Anmeldung: **17.02.2010**
(21) Anmeldenummer: 09167869.8
(22) Anmeldetag: 14.08.2009
(51) Int. Cl.: A61F 13/00, D04B 1/16, D04B 21/16, A61L 15/28, A61L 15/60, D03D 15/00

(54) **Wundpflegeartikel, aufweisend Textilbänder mit Fasern mit gelbildenden Eigenschaften sowie Fasern mit nicht gelbildenden Eigenschaften**

(30) Priorität: 15.08.2008 DE 102008037888
(71) Anmelder: Riesinger, Birgit, 48346 Ostbevern (DE)
(72) Erfinder: Riesinger, Birgit, 48346 Ostbevern (DE)
(74) Vertreter: Storz, Ulrich

(57) **Zusammenfassung**

Gegenstand der Erfindung ist ein Wundpflegeartikel, aufweisend mindestens ein flächiges Gebilde enthaltend Fasern mit gelbildenden Eigenschaften, sowie Fasern mit nicht gelbildenden Eigenschaften, dadurch gekennzeichnet ist, dass die Fasern in Form von flächigen Textilbändern angeordnet sind.

## Beschreibung

Die Erfindung betrifft einen Wundpflegeartikel gemäß dem Oberbegriff des Anspruchs 1.

Ein solcher Wundpflegeartikel eignet sich insbesondere zur Aufnahme von Exsudat aus chronischen Wunden, wie z.B. bei Diabetes, Ulcus cruris und ähnlichen Erkrankungen auftreten.

Der Begriff "Exsudat" bezeichnet eine über die entzündlichen Prozesse des Wundödems vom Blutplasma abgeleitete Wundflüssigkeit. So wie das Blut für den Transport von Nährstoffen und anderen Botenstoffen und damit für die Versorgung verschiedener Teile des Körpers verantwortlich ist, dient das Exsudat auf ganz ähnliche Weise der Versorgung des Wundbettes und der darin ablaufenden Heilungsprozesse. Um dieser Vielzahl an Funktionen gerecht zu werden, enthält es ein breites Spektrum an Komponenten, woraus ein spezifisches Gewicht resultiert, das leicht oberhalb dessen von Wasser liegt. Darin unterscheidet es sich auch vom Transsudat, welches von nicht-entzündlichen Prozessen abgeleitet ist und ein deutlich geringeres spezifisches Gewicht mit einem geringen Zell- und Proteingehalt aufweist. Neben der Bereitstellung von Nährstoffen für die Fibroblasten und Epithelzellen koordiniert das Exsudat die verschiedenen Prozesse der Wundheilung zeitlich und räumlich durch seinen hohen Gehalt an Wachstumsfaktoren und Zytokinen. Diese werden vor allem durch Thrombozyten, Keratinozyten, Makrophagen und Fibroblasten gebildet. Sie beeinflussen die Motilität, Migration und Proliferation der verschiedenen an der Wundheilung beteiligten Zellen. So wird das Einwandern von Zellen in den Wundgrund ebenso gefördert wie die Versorgung des neugebildeten Granulationsgewebes durch die Angiogenese. Auch die Wundreinigung wird durch das Exsudat unterstützt. Es enthält verschiedene Serin-, Cystein- und Aspartatproteasen sowie Matrix-Metalloproteasen, die in ihrer Aktivität streng reguliert irreversibel geschädigtes Gewebe abbauen und somit das Wundbett für die nachfolgenden Phasen der Heilung vorbereiten.

Bestandteile des physiologischen Exsudats sind insbesondere Salze, Glucose, Zytokine und Wachstumsfaktoren, Plasmaproteine, Proteasen (insbesondere Matrix-Metalloproteasen), Granulozyten und Makrophagen.

Kommt es nicht innerhalb einiger Wochen zu einer deutlichen Progression des Wundheilungsverlaufs entsprechend der verschiedenen Phasen der Wundheilung, so spricht man von einer chronischen Wunde. Dabei betrachtet man jedoch bereits länger als drei Tage andauernde exsudative Phasen als Komplikation und spricht von einer pathologischen Exsudation, welche zu einer Chronifizierung der Wunde beitragen kann. Die zugrunde liegenden Ursachen sind meist komplex und können durchaus auch systemischer Natur sein. Es überrascht jedoch aufgrund der zuvor erläuterten Bedeutung des Exsudats für die Wundheilung nicht, dass sich Komplikationen der Wundheilung in einer deutlich veränderten Zusammensetzung und Wirkung des Exsudats widerspiegeln.

Unter anderem durch eine Konzentrationsverschiebung der einzelnen Bestandteile des Exsudats verliert das normalerweise heilungsfördernde Exsudat bei chronischen Wunden seine positive Wirkung. Insbesondere der Gehalt an inflammatorischen Zytokinen und Proteasen ist in pathologischem Exsudat signifikant erhöht. Der Gehalt an Wachstumsfaktoren ist dagegen verringert. Ein besonders gravierender Unterschied ergibt sich hinsichtlich der Aktivität der zuvor angesprochenen Matrix-Metalloproteasen. Neben der Vorbereitung des Wundbetts sind diese auch beim späteren Umbau des Granulations- zum Narbengewebes beteiligt. Diese Enzyme werden normalerweise als inaktives Präenzym gebildet und in ihrer Aktivierung durch entsprechende Inhibitoren reguliert (tissue inhibitors of metalloproteases, TIMPs), welche gleichzeitig selbst eine positive Wirkung auf das Zellwachstum haben. Im chronischen Exsudat scheint aufgrund von Störungen in diesem Regulationssystem die Aktivität der Proteasen erhöht, was möglicherweise zu einer aktiven Wundregression beiträgt. Das pathologische Exsudat ist hinsichtlich des Gehalts seiner Komponenten aus dem der Wundprogression förderlichen Gleichgewicht geraten. Daraus ergeben sich verschiedene Komplikationen, die zur weiteren Verschlechterung und Chronifizierung der Wunde beitragen.

In der Wundversorgung finden seit jüngerer Zeit gelbildende Polymere verstärkt Verwendung. Diese weisen einerseits eine hohe Bindungskapazität für die genannten Exsudate auf, bilden aber gleichzeitig eine nicht haftende und angenehm kühlende Geloberfläche aus. Besonders häufig wird hier Carboxymethycellulose (CMC) verwendet.

Carboxymethycellulosen sind Derivate der Cellulose, bei denen ein Teil der Hydroxylgruppen der Cellulose als Ether mit einer Carboxymethyl-Gruppe verknüpft sind. Bei der Herstellung wird die Cellulose beispielsweise in reaktivere Alkalicellulose überführt und anschließend mit Chloressigsäure zur Carboxymethylcellulose umgesetzt. Die Cellulose-Struktur bleibt erhalten. In der Säureform ist CMC unlöslich in Wasser. Unter alkalischen Bedingungen sind sie hingegen relativ gut in Wasser löslich.

CMC kann bis zum ca. vierfachen des eigenen Volumens an Flüssigkeit in sich aufnehmen, bevor es zur Kolloidbildung kommt. Die Flüssigkeitsaufnahme erfolgt anders als bei Alginaten, bei denen Ionenaustauschvorgänge stattfinden, schnell, wobei die Flüssigkeit und darin gelöste Bestandteile fest eingebunden werden, bis die Faser übersättigt ist.

Carboxymethylcellulose liegt insbesondere in Form von Natriumcarboxymethylcellulose vor. In Hygiene- und Wundprodukten werden die Fasern in eine flächige Matrix überführt. Durch die Aufnahme von Flüssigkeit aus dem Wundexsudat werden die Fasern nach und nach in ein Gelkissen umgewandelt, das die Flüssigkeit hält und nicht wieder freigibt. Dabei sind die Fasern so aufgebaut, dass das Wundexsudat nur in vertikaler Richtung aufgenommen wird. Dies bedeutet dass, solange die Kapazität reicht, das Exsudat nicht über den Wundrand fließt. Auf diese Weise kann eine Wundrandmazeration effektiv verhindert werden.

Die Verwendung von Wundpflegeartikeln aufweisend CMC eignet sich darüber hinaus insbesondere zur/zum
- Behandlung von stark exsudierenden Wunden
- Unterstützung der Wundheilungsvorgänge durch Erhaltung eines feuchten Milieus
- Hämostatische Eigenschaften

### Stand der Technik

Aus der EP12166319 ist eine Wundauflage bekannt, bei der ein gelbildendes Garn, beispielsweise aus Carboxymethylcellulose (CMC), mit einem nicht gelbildenden Garn, beispielsweise Nylon, verflochten ist, wobei letzteres als stabilisierendes Garn dient, um die strukturelle Integrität der Wundauflage auch unter Feuchtigkeitseinfluss zu gewährleisten.

Aus der DE 69530180 ist ein nicht haftender Wundverband in Bahnenform bekannt, der von 50 Gew.-% bis 95 Gew.-% textile Fasern, gemischt mit von 5 Gew.-% bis 50 Gew.-% gelbildende Fasern aufweist. Die in der Schrift genannten textilen Fasern, beispielsweise Cellulosefasern weisen eine relativ schwache, dagegen die faserigen Polymere eine erhöhte Absorptionsfähigkeit auf, die bis zum 50-fachen des Eigengewichtes reichen soll. Nachteilig bei dem bekannten Wundverband ist, dass sowohl die textilen als auch die gelbildenden Fasern eine für bestimmte Anwendungen, beispielsweise für sehr stark exsudierende Wunden unzureichende Aufsaugkapazität aufweisen. Dieser Wundverband weist daher nur eine geringe Aufnahmekapazität für Wundexsudate auf. Überdies ist das Aufnahmeverhalten nur schlecht dosierbar.

Verfahren zur chemischen Modifikation von Cellulose dergestalt, dass diese gelbildende Eigenschaften aufweist, sind aus dem Stand der Technik bekannt.

So ist generell ein Verfahren zur Carboxyalkylierung von Cellulose bekannt, das insbesondere zur Herstellung von Carboxymethylcellulose führt. Dabei wird Cellulose (unverzweigtes Polymer aus 1-4-β-glykosidisch verknüpften Glucosemolekülen mit einer Kettenlänge zwischen einhundert und zehntausend Monomeren) durch alkali-katalysierte Reaktion mit einer Chloralkansäure (beispielsweise Chloressigsäure) umgesetzt. Die polaren CarboxylGruppen machen die Cellulose löslich und sind für die Gelbildung verantwortlich. Die Funktionseigenschaften von CMC hängen vom Substitutionsgrad der Cellulose ab, d.h. von der Anzahl an Carboxymethylgruppen pro Glucoseuntereinheit.

Nachteil dieser Verfahren ist, dass sich - wenn die Einhaltung genau kontrollierter Verfahrensbedingungen gefragt ist, wie es für die genaue Einstellung eines homogenen Substitutionsgrades und damit der gelbildenden Eigenschaften erforderlich ist - sich dieses Verfahren nicht für flächige Cellulosegebilde anwenden lässt, sondern lediglich für dispergierte Cellulosefasern oder für sehr schmale Cellulosebänder. Dies gilt auch für die Wundauflagen gemäß den oben genannten, den Stand der Technik bildenden Dokumenten.

So lassen sich mit diesem Verfahren schlauchförmige Cellulosegewebe bzw. Gestricke mit einem Schlauchdurchmesser von maximal 5 cm kontrolliert carboxymethylieren. Ein so hergestelltes Produkt ist unter dem Markennamen "Rapid Rhino Sinu Knit" im Handel erhältlich. Wollte man mit diesem Verfahren größere Gebilde herstellen, so stiege der verfahrenstechnische Aufwand, der erforderlich ist, um weiterhin einen homogenen Substituierungsgrad zu gewährleisten, überproportional stark an. Aus diesem Grunde ist es ökonomisch nicht sinnvoll, größere Gebilde mit dem genannten Verfahren zu behandeln.

Flächige Gebilde, die wie sie z.B. für die Verwendung als großflächiger Wundpflegeartikel erforderlich sind (z.B. 10 x 10 cm), lassen sich mit den aus dem Stand der Technik bekannten Verfahren nicht carboxymethylieren.

### Aufgabe der vorliegenden Erfindung

Aufgabe der vorliegenden Erfindung ist es, eine Wundauflage bereitzustellen, die die genannten Nachteile nicht aufweist. Diese Aufgabe wird mit den Merkmalen der unabhängigen Ansprüche gelöst.

Demnach ist ein Wundpflegeartikel, aufweisend mindestens ein flächiges Gebilde enthaltend Fasern mit gelbildenden Eigenschaften, sowie Fasern mit nicht gelbildenden Eigenschaften, vorgesehen, der dadurch gekennzeichnet ist, dass die Fasern in Form von flächigen Textilbändern angeordnet sind.

Der Begriff "Fasern", wie er hier verwendet wird, bezieht sich
a) sowohl auftextile Einzelfasern (sogenannte Monofilamente), als auch
b) aus diesen Monofilamenten hergestellte Polyfilamente, insbesondere versponnene, verzwirnte oder vertwistete Garne.

Dabei liegen die besagten Monofilamente bevorzugt nicht in versponnener, verzwirnter oder vertwisteter Form vor - also beispielsweise als Garn - sondern werden direkt als Monofilamente zu den besagten Textilbändern verarbeitet.

Der Begriff "flächiges Textilband", wie hier verwendet, bezieht sich auf ein flächiges Band, das aus Fasern im obigen Sinne besteht. Insbesondere kann hier vorgesehen sein, dass ein Textilband
a) nur aus Fasern mit nicht gelbildenden Eigenschaften,
b) nur aus Fasern mit gelbildenden Eigenschaften, oder
c) sowohl aus Fasern mit nicht gelbildenden Eigenschaften als auch aus Fasern mit gelbildenden Eigenschaften
besteht.

Die besagten Textilbänder weisen bevorzugt Breiten im Bereich zwischen einschließlich 1 mm und einschließlich 8 cm auf, besonders bevorzugt zwischen einschließlich 5 mm und einschließlich 3 cm.

Bevorzugt ist vorgesehen, dass der besagte Wundpflegeartikel mehrere flächige Textilbänder aufweist, die miteinander verwoben, verflochten, verwirkt oder verknüpft sind. Beispiele für diese Ausführungsformen sind in den Abbildungen gezeigt.

Auf diese Weise kann eine flächige Anordnung mit beliebig dimensionierten Flächenmaßen hergestellt werden, die eine griffige, rauhe Struktur aufweist, die vom Patienten als angenehm empfunden wird. Hinzu kommt, dass z.B. durch entsprechende räumliche Anordnung der Bänder (beispielsweise bei Ausbildung einer Zopfform, siehe Abbildungen) die besagte Anordnung eine dreidimensionale Struktur aufweisen kann, was ebenfalls vom Patienten als angenehm empfunden wird.

Hier kann insbesondere vorgesehen sein, dass bei der erwähnten Verarbeitung (Verweben, Verflechten, Verwirken, Verknüpfen) Durchlässe zwischen den Bändern belassen sind. Dies ist beispielsweise dann wichtig, wenn die genannte flächige Anordnung als Hülle um einen Absorptionskörper verwendet wird, der stark absorbierende Substanzen wie z.B. Superabsorbierende Polymere enthält. Es wird durch die Durchlässe verhindert, dass die Anordnung, die bei Kontakt mit Flüssigkeit partiell ein Gel ausbildet, für Flüssigkeiten unpassierbar wird. Stattdessen wird durch die Durchlässe gewährleistet, dass selbst in feuchtem Zustand Flüssigkeit die Anordnung passieren und in den Absorptionskörper gelangen kann.

Alternativ kann vorgesehen sein, dass in den besagten Textilbänder in Längsrichtung mindestens ein elastisches Band - beispielsweise ein starkes Elasthanband - angeordnet, dergestalt, dass die Bänder eine plissierte und/oder geraffte Struktur annehmen.

Grundsätzlich kann vorgesehen sein, dass
a) die Textilbänder durchweg sowohl aus Fasern mit nicht gelbildenden Eigenschaften als auch aus Fasern mit gelbildenden Eigenschaften bestehen, oder dass
b) die Textilbänder nur aus Fasern mit nicht gelbildenden Eigenschaften mit Textilbändern nur aus Fasern mit gelbildenden Eigenschaften verwoben sind.

Bevorzugt ist dabei vorgesehen, dass die Fasern mit gelbildenden Eigenschaften auf chemisch modifizierter Cellulose basieren.

Bei Cellulose handelt es sich um ein unverzweigtes Polymer aus 1-4-β-glykosidisch verknüpften Glucosemolekülen mit einer Kettenlänge zwischen einhundert und zehntausend Monomeren. Formel 1 zeigt exemplarisch einen Ausschnitt aus einem Cellulose-Molekül.

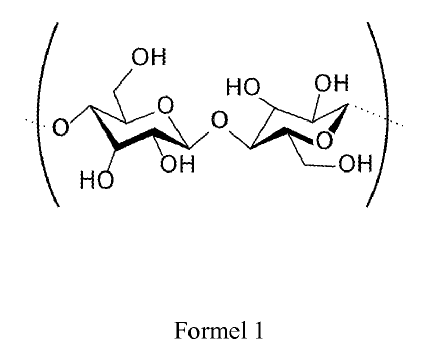

Ebenfalls unter den Begriff "Cellulose" fällt im Sinne der vorliegenden Erfindung die bekannte Viskose (Cellulose-Xanthogenat, "Rayon"). Viskose wird durch Behandlung von Cellulose mit 18 - 22-%iger Natronlauge hergestellt, wobei sich zunächst Alkalicellulose bildet, das Natriumsalz der Cellulose. Die Alkalicellulose wird gepresst und zerfasert. Im nächsten Arbeitsschritt wird die Alkalicellulose mit Schwefelkohlenstoff umgesetzt, wobei Cellulosexanthogenat entsteht. Hierbei erfolgt eine Substitution an eine oder mehrere OH-Gruppen der Glucose-Monomere. Um Viskose zur Herstellung von Fasern zu erhalten, muss dabei im Durchschnitt auf zwei Glucosemonomere ein Schwefelkohlenstoff-Substituent kommen. Anschließend wird eine sogenannte "Spinnlösung" hergestellt. Hierfür wird das erhaltene Cellulosexanthogenat in 7%-iger Natronlauge gelöst, entgast und durch feine Düsen in eine Lösung von Schwefelsäure und Sulfaten gepresst. Darin werden die Schwefelkohlenstoffmoleküle, die an die Cellulose gebunden sind, zum größten Teil wieder abgespalten, und es entsteht die Viskosefaser. Ein Ausschnitt aus einem Viskose-Molekül ist beispielhaft in Formel 2 gezeigt.

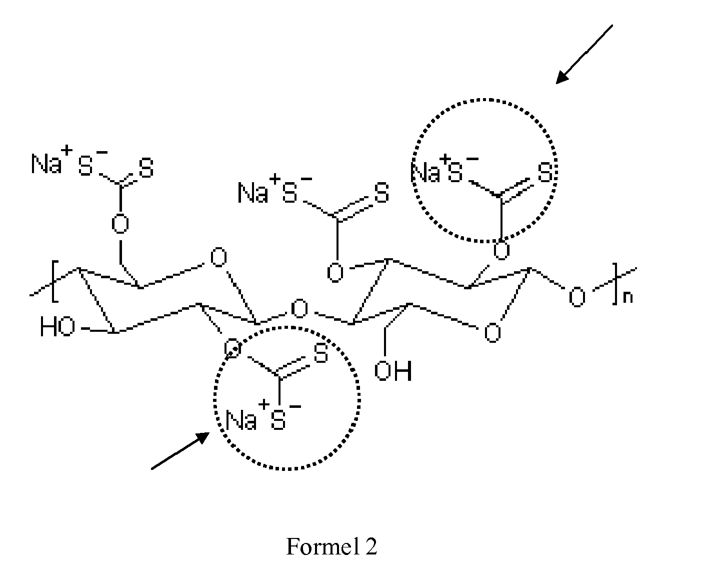

Die Pfeile in Formel 2 verweisen auf die Xanthogenat-Substituenten; diese sind übertrieben häufig dargestellt, da bei Viskose wie bereist gesagt im Durchschnitt auf zwei Glucosemonomere ein Schwefelkohlenstoff-Substituent kommt.

Weitere Stoffe, die im Sinne der vorliegenden Erfindung unter den Begriff "Cellulose" fallen, sind
- Lyocell (ein aus Cellulose künstlich hergestellter Faserwerkstoff, der durch Lösung von Cellulose in einem Lösungsmittel, im Regelfall N-Methylmorpholin-N-oxid, und anschließendes Spinnen ("solvent spinning") hergestellt wird, und
- Polynosic (aus regenerierter Cellulose nach einem modifizierten Viskosespinnverfahren hergestellte Fasern, die im Vergleich zu Viskose eine höhere Nassfestigkeit, höhere Alkalibeständigkeit und ein geringeres Quellvermögen aufweisen).

Die Fasern mit nicht gelbildenden Eigenschaften weisen bevorzugt chemisch modifizierte Cellulose auf.

Bevorzugt handelt es sich bei der chemisch modifizierten Cellulose um zumindest partiell substituierte Cellulose, bevorzugt Celluloseether, wie z.B. alkylierte Cellulose (z.B. Methylcellulose, Ethylcellulose, Propylcellulose), hydroxialkylierte Cellulose (z.B. Hydroximethyl-Cellulose, Hydroxiethyl-Cellulose, Hydroximethyl-Cellulose, Hydroxipropylmethyl-Cellulose) oder carboxyalkylierte Cellulose (z.B. Carboxymethylcellulose, Carboxyethylcellulose, Carboxypropylcellulose).

Bevorzugt ist vorgesehen, dass die modifizierte Cellulose einen Substitutionsgrad zwischen einschließlich 0,05 und einschließlich 3 Alkyl-, Hydroxialkyl- oder Carboxyalkylgruppen pro Glucoseeinheit aufweist. Dabei bezeichnet ein Substitutionsgrad von 3 drei Substituenten pro Glucoseeinheit, während ein Substitutionsgrad von 0,05 einen Substituenten pro 2000 Glucoseeinheiten bezeichnet.

Besagte chemisch modifizierte Cellulosen gehen bei Kontakt mit Wasser sukzessive in einen gelartigen Zustand über. Dabei ist die gelbildende Eigenschaft abhängig vom Substitutionsgrad der Cellulose, d.h. je höher der Substitutionsgrad, desto früher tritt die Gelbildung ein. Mit Hilfe des Substitutionsgrades lässt sich jedoch auch der Eintritt des Verlustes der strukturellen Integrität der chemisch modifizierten Cellulose steuern. Hiermit ist gemeint, dass die Gelbildung der chemisch modifizierten Cellulose im äußersten Fall dazu führt, dass die chemisch modifizierte Cellulose in ein vollständig amorphes und nicht mehr als eine strukturell integere Einheit zu bezeichnendes Gel überführt wird, dass sich unter Umständen nicht mehr als Einheit aus der Wunde entfernen lässt, sondern z.B. mit Hilfe eines Spatels abgeschabt werden muss.

Der Verlust der strukturellen Integrität tritt bei einer hoch substituierten Cellulose relativ früh ein, während er bei einer relativ niedrig substituierten Cellulose relativ spät oder gar nicht eintritt.

Ebenso kann es sich bei der chemisch modifizierten Cellulose um oxidierte Cellulose handeln. Eine solche oxidierte Cellulose ist z.B. aus der EP1215217 bekannt. Sie weist ähnliche Eigenschaften auf wie carboxyalkylierte Cellulose, lässt sich jedoch kostengünstiger und überdies aus recyceltem Material herstellen.

Generell gilt, dass die oben genannten Verfahren zur chemischen Modifikation von Cellulose auch auf Viskose anwendbar sind. Wie oben bereits erläutert, weist Viskose im Durchschnitt auf zwei Glucosemonomere einen Schwefelkohlenstoff-Substituenten auf. Es sind also noch ausreichend freie OH-Gruppen vorhanden, die in oben genannter Weise substituiert werden können, bevorzugt carboxyalkyliert (-methyliert) oder oxidiert.

Bei der chemisch modifizierten Cellulose handelt es sich bevorzugt um Carboxyalkylcellulose oder Carboxyalkylviskose. Formel 3 zeigt exemplarisch einen Ausschnitt aus einem Carboxymethylcellulose-Molekül.

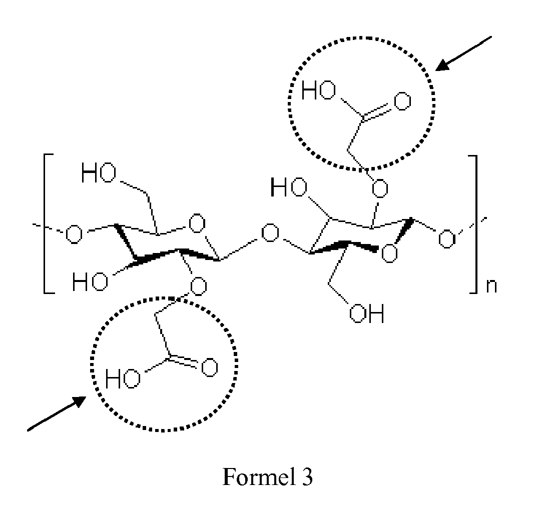

Die Pfeile verweisen auf die Substituenten, bei denen es sich um Carboxymethylgruppen (-CH₂-COOH) handelt. Der Substitutionsgrad beträgt hier 1 pro Glucoseeinheit.

Auch hier sei darauf hingewiesen, dass im Sinne der Erfindung andere OH Gruppen durch Xanthogenat-Substituenten substituiert sein können. In diesem Falle handelt es sich bei besagtem Molekül um Carboxymethyl-Viskose, ebenso denkbar sind ähnlich substituierte Fasern auf Basis von Lyocell oder Polynosic.

Wie bereits erwähnt, kann der Substitutionsgrad erfindungsgemäß im Bereich von einschließlich 0,05 bis einschließlich 3 liegen. Bei den Substituenten kann es sich in Abweichung von dem oben gesagten auch um andere Alkyl-, Hydroxialkyl- oder Carboxyalkylgruppen handeln; in diesem Fall handelt es sich dann um die entsprechenden analog modifizierten Cellulosen.

Bevorzugt ist weiterhin vorgesehen, dass die Fasern mit nicht gelbildenden Eigenschaften trotz Einfluss von Feuchtigkeit ihre strukturelle Integrität aufrecht erhalten.

Diese Fasern bilden insbesondere kein Gel aus, wenn sie mit Feuchtigkeit in Berührung kommen. Bevorzugt weisen diese Fasern mindestens einen Fasertyp ausgewählt aus der Gruppe enthaltend Cellulose, Viskose, Leinen, Wolle, und/oder synthetische Fasern, wie z.B. Polyamidfasern ("Nylon"), Polyesterfasern, Polyacrylfasern, Polypropylenfasern, Elasthanfasern ("Lycra") und dergleichen auf.

Es muss hier noch einmal klar unterscheiden werden zwischen nicht chemisch modifizierter Cellulose (oder Viskose), die keine gelbildenden Eigenschaften aufweist, und gemäß obiger Beschreibung chemisch modifizierter Cellulose (oder Viskose), die gelbildende Eigenschaften aufweist.

Die besagten nicht gelbildenden Fasern haben hier also im wesentlichen eine stützende Funktion, die verhindert, dass sich der Wundpflegeartikel bei Kontakt mit Flüssigkeit (beispielsweise Wundexsudat) vollständig in ein Gel umwandelt und sich dann nicht mehr als ganzes von/aus der Wunde entfernen lässt. Sie gewährleisten also die Aufrechterhaltung der strukturellen Integrität des erfindungsgemäßen Wundpflegeartikels. Dies ist insbesondere deswegen wichtig, da auf diese Weise auch in die Gelform überführte Bestandteile des Wundpflegeartikels zusammen mit letzterem wieder aus der Wunde entfernt werden können.

Wichtig für letzteres ist insbesondere, dass - wie erfindungsgemäß vorgesehen - gelbildende Fasern und nicht gelbildende Fasern in unmittelbarem räumlichem Zusammenhang zueinander angeordnet sind.

Bevorzugt ist ferner vorgesehen, dass der Anteil an Fasern mit gelbildenden Eigenschaften an besagtem flächige Gebilde des Wundpflegeartikels im Bereich zwischen einschließlich 50 Gew.-% und einschließlich 100 Gew.-% liegt.

Besonders bevorzugt ist vorgesehen, dass das besagte flächiges Gebilde aufweisend flächige Textilbänder
a) eine Hülle um einen absorbierenden Körper ausbildet,
b) die Form eines Tupfers ausbildet, und/oder
c) die Form eines Wundtuchs, einer Wundauflage, einer Wundkompresse, eines Wundpolsters, einer Bandage oder eines Strumpfes ausbildet.

Weiterhin ist bevorzugt vorgesehen, dass der Wundpflegeartikel außerdem Superabsorbierende Polymere aufweist.

Superabsorbierende Polymere (SAP) sind Kunststoffe, die in der Lage sind, ein Vielfaches ihres Eigengewichts - bis zum 1000-fachen - an Flüssigkeiten aufzusaugen. Chemisch handelt es sich dabei um ein Copolymer aus Acrylsäure (Propensäure, C₃H₄O₂) und Natriumacrylat (Natriumsalz der Acrylsäure, NaC₃H₃O₂), wobei das Verhältnis der beiden Monomere zueinander variieren kann. Zusätzlich wird ein so genannter Kernvernetzer (Core-Cross-Linker, CXL) der Monomerlösung zugesetzt, der die gebildeten langkettigen Polymermoleküle stellenweise untereinander durch chemische Brücken verbindet (sie "vernetzt"). Durch diese Brücken wird das Polymer wasserunlöslich. Beim Eindringen von Wasser oder wässrigen Salzlösungen in die Polymerpartikeln quellen sie auf und straffen auf molekularer Ebene dieses Netzwerk, so dass das Wasser ohne Hilfe nicht mehr entweichen kann.

Die Superabsorber-Teilchen können in Pulver- oder Granulatform von einer Partikelgröße zwischen 100 und etwa 1000 µm vorliegen.

Bevorzugt handelt es sich bei den Superabsorbierenden Polymeren um Polymere in Faser-, Garn-, Watte- Vlies- oder Gewebeform. In einer anderen bevorzugten Ausführungsform handelt es sich bei den Superabsorbierenden Polymeren um Polymere in Pulver- oder Granulatform.

Weiterhin ist bevorzugt vorgesehen dass der Wundexsudate absorbierende Körper mindestens ein Material aufweist, das ausgewählt ist aus der Gruppe enthaltend
a) eine Fasermatte, insbesondere aus einem Airlaid, mit eingearbeiteten Superabsorbierenden Polymeren,
b) ein Vlies, ein Nonwoven, ein Airlaid, eine Matte, ein Gewirke oder ein Gewebe aufweisend Fasern oder Garne aus Superabsorbierenden Polymeren,
c) ein absorbierendes Cellulosematerial
d) eine lose Füllung aufweisend Superabsorbierende Polymere, und/oder
e) eine Matte aus einem Weichschaumstoff.

Wesentliche Vorteile dieser Konfiguration sind
- dass die Hülle selbst erhebliche Mengen an Wundexsudaten aufnehmen kann, so dass sich das gesamte Aufnahmevermögen des Wundpflegeartikels erhöht;
- die Hülle durch den Anteil an gelbildenden Fasern eine geringe Haftung an der Wunde aufweist;
- die Hülle durch den Anteil an stützenden Fasern nahtfest ist,
- die Hülle bei Verwendung von gelbildenden Fasern einen angenehmen kühlenden und rückfeuchtenden Effekt auf die Wunde ausübt;
- die stützenden Fasern die Integrität der Hülle auch bei paralleler Verwendung von gelbildenden Fasern gewährleisten; und
- ein Absorptionsgefälle zwischen innen gelegenem absorbierenden Körper und Hülle entsteht, wobei der innere absorbierende Körper als Reservoir dient und eine vollständige Desintegrierung der Hülle ausbleibt, weil überschüssige Flüssigkeit nach innen abgeleitet wird.

Der Begriff "Airlaid" bezeichnet einen speziellen Vliesstoff aus Zellstoff und Polyolefinfasern, in den ggf. Superabsorbierende Polymere eingebettet sind. Er kann in Mattenform ausgebildet und bevorzugt eine flüssigkeitsdurchlässige Hülle aufweisen, die z.B. aus Polypropylen besteht.

Die Superabsorbierenden Polymere können dabei in Faser-, Garn-, Watte- Vlies- oder Gewebeform, in Pulver- oder Granulatform, oder in Form eines Schaums, einer Schüttung, eines Presslings oder einer Schüttung aus Schnipseln bestehend aus einer zerschnittenen Airlaidmatte vorliegen.

In einer weiteren bevorzugten Ausgestaltung ist vorgesehenen, dass der Wundexsudate absorbierende Körper mindestens ein Material aufweist, das ausgewählt ist aus der Gruppe enthaltend eine Matte, insbesondere aus einem Airlaid, mit eingearbeiteten Superabsorbierenden Polymeren, und/oder eine lose Füllung aus Superabsorbierenden Polymeren. Besagte Airlaidmatte ist bereits oben beschrieben und kann bevorzugt einen im wesentlichen flachen Materialabschnitt aus Absorptionsmaterial aufweisen, der z. B. aus einem aufsaugenden Vlies aus den genannten Fasern mit darin verteilten Superabsorbierenden Polymeren besteht.

Dieser Wundexsudate absorbierende Körper kann der absorbierenden Einlage entsprechen, die in einer Wundauflage der Anmelderin der vorliegenden Erfindung enthalten ist, wie sie beispielsweise in der WO03094813, der WO2007051599 und der WO0152780 offenbart ist und unter dem Handelsnamen "sorbion sachet" vertrieben wird. Der Offenbarungsgehalt der genannten Schriften sei dem Offenbarungsgehalt dieser Schrift vollumfänglich beigefügt.

Der Wundexsudate absorbierende Körper kann in einer anderen Ausgestaltung ebenso einen Kern bilden, der aus - ggf. flockenartiger - Cellulose und/oder Cellulosederivaten, bevorzugt Flockenzellstoff oder Zellwollfasern, Superabsorbierenden Polymeren in Granulatform sowie einem Klebstoff besteht, wobei die Granulate an die Cellulose bzw. den Zellstoff in mehreren Höhen angeklebt sind, und die Granulate über mehr als 50 % der gesamten Bauhöhe wenigstens eines Abschnitts des Kerns verteilt sind, wobei vermengte Bereiche von Granulat und Cellulose und/oder Cellulosederivaten vorliegen. Der Gewichtsanteil der Superabsorbierenden Polymere kann dabei bevorzugt im Bereich zwischen 10 - 25 Gew.-% liegen. Ähnliche Konstruktionen sind aus herkömmlichen Inkontinenzmaterialien bekannt und bei Hygienebinden für ihre polsternden Eigenschaften bekannt.

Um besagten Kern herum kann eine Hülle angeordnet sein, die in Bereichen überlappend angeordnet ist, und der z.B. eine Klebenaht überdeckt bzw. Teil derselben ist.

Ebenso kann innerhalb der Hülle ein Abschnitt eines hydrophoben und/oder wasserabweisenden bzw. wasserundurchlässigen Materials vorgesehen sein, der Durchnässungs- oder Wäscheschutz fungiert.

Der Wundexsudate absorbierende Körper kann in einer anderen Ausgestaltung ebenso mindestens eine flache Lagen aus einem Zellstoff enthalten, an welche Superabsorbierende Polymere - bevorzugt in Granulatform - geklebt sind. Dadurch ergibt sich in einer bevorzugten Ausgestaltung ein Aufbau des Körpers, der wenigstens drei Schichten aufweist, wobei zwei Deckschichten eine Schicht aufweisend Superabsorbierende Polymere umgeben.

Dabei liegen in der Ebene keine Vermengungen von Zellstoff und Superabsorbierenden Polymeren vor; sondern lediglich fixierte Benachbarungen beider Materialien. Die ggf. vorgesehenen mehreren lagen können dabei in einer bevorzugten Ausgestaltung auch durch Walzen, Pressen, Kalandrieren oder ähnliche Verfahren physisch miteinander verdichtet sein.

Überdies kann der Körper sich wiederholende Musterungen oder Maserungen aufweisen, wie z.B. ein Karomuster, ein Stanzmuster oder dergleichen aufweisen.

Der besagte Wundexsudate absorbierende Körper kann überdies ggf. eine Hülle aus einem durchlässigen Material aufweisen; diese kann in ihren Randbereichen unterschiedliche Verbindungen bzw. Nähte aufweisen, die insbesondere durch Klebungen erzeugt sind. So kann z.B. vorgesehen sein, dass der Verbindungsbereich zwischen dein beiden Seiten der Hülle an mindestens einer Seite des Wundexsudate absorbierenden Körpers - bevorzugt in Längsrichtung - schmaler ausgestaltet ist als an mindestens einer anderen Seite, so dass sich im ersteren Falle, anders als im letzteren Falle ein sich auffaltbarer Überstand ergibt.

Besagte Hülle kann bevorzugt aus einem Nonwoven aus Polypropylen mit einem Flächengewicht von 10 - 40 g/m² bestehen.

Bevorzugt kann überdies vorgesehen sein, dass der so beschriebene Wundexsudate absorbierende Körper an wenigstens einer Innerseite der ihn umgebenden Hülle fixiert ist, bevorzugt durch Klebungen.

In einer anderen Ausführungsform ist bevorzugt vorgesehen, dass es sich bei dem Weichschaumstoff der Hülle um mindestens ein Material ausgewählt aus der Gruppe enthaltend thermoplastische Weichschäume, wie Polyurethan-, Polyamid- oder Polyetherschaum, Silikonschaum sowie Cellulose-Schaum oder Naturschwamm handelt.

Naturschwämme beispielsweise der Klasse der Hornkieselschwämme (Demospongiae) weisen ähnlich wie technische Schaumstoffe ein Absorptionsvermögen für Flüssigkeiten auf. Überdies weisen sie wachstumshemmende Eigenschaften gegenüber Mikroorganismen auf, um sich vor dem Ansiedeln von sessilen Organismen zu schützen. Diese Eigenschaften können auch in Zusammenhang mit der Wundversorgung sinnvoll sein, um Bakterienwachstum in der Wundauflage und/oder in der Wunde zu verhindern. Ebenso weisen diese Schwämme wachstumshemmende Eigenschaften gegenüber Pilzen und Einzellern auf. Hinzu kommt, dass solche Schwämme Flüssigkeiten zu absorbieren imstande sind und sich daher für die Aufnahme von Exsudaten hervorragend eignen.

Besagter Naturschwamm kann in dünnen Scheiben, die z. B. durch thermisches Schneiden erzeugt worden sind, auf die Wunde aufgelegt sein.

Der Weichschaumstoff kann ggf. mehrlagig ausgestaltet sein, wobei die einzelnen Lagen bevorzugt Dicken zwischen 0,5 mm und 10 mm aufweisen können.

Der Weichschaumstoff kann offenzellig und geschlossenzellig ausgestaltet sein. Überdies kann der Weichschaumstoff auch als Integralschaum ausgestaltet sein.

In besagter Ausführungsform ist besonders bevorzugt vorgesehen, dass die Hülle aus stützenden Fasern und hydroaktiven Polymeren Kanäle, Lochungen oder Stanzungen aufweist. Diese erleichtern den Durchtritt von Flüssigkeit, insbesondere Exsudat, zum zentral gelegenen absorbierenden Körper. Diese Ausgestaltung ist insbesondere bei Verwendung von CMC als hydroaktives Polymer vorteilhaft. CMC schrumpft bei Kontakt mit Flüssigkeit, was zu einer Vergrößerung besagter Löcher führt und so den Flüssigkeitsdurchtritt überdies erleichtert.

Einzelne Bestandteile der Hülle können an den Rändern der Wundauflage miteinander physikalisch verbunden sein, z.B. durch Verkleben, Vernähen oder Verschweißen. Weitere physikalische Verbindungstechniken sind hier denkbar und im Sinne der Erfindung erfasst. Als Schweißverfahren kommt insbesondere thermisches Schweißen oder Ultraschallschweißen in Frage.

Grundsätzlich wird beim Verbinden zweier Lagen, insbesondere beim Verkleben, Vernähen oder Verschweißen eine außen liegende Naht erzeugt. Diese kann in einigen Verwendungsfällen nachteilig sein, so z.B. dann, wenn der Wundpflegeartikel in eine Wundtasche eingelegt wird, da hier die Nahtüberstände Reibekanten darstellen, die ggf. zu Entzündungen, zumindest aber zu Schmerzen führen können. Für solche Fälle kann vorgesehen sein, dass die Bestandteile der Hülle durch eine innen liegende Naht verbunden sind; eine solche innen liegende Naht kann z.B. erzeugt werden, in dem man zwei rechteckige Lagen der Hülle an drei Seiten auf herkömmliche Weise miteinander vernäht, und das erhaltene Produkt dann "umkrempelt". In den so gebildeten Innenraum kann dann eine Lage enthaltend Superabsorbierende Polymere entsprechend gemäß der obigen Beschreibung eingebracht werden. Durch das Umkrempeln wird überdies bewirkt, dass die Ecken des Wundpflegeartikels abgerundet (somit gleichsam "entschärft") werden und der Wundpflegeartikel insgesamt eine gerundete Form annimmt, die unter Umständen z.B. einem Tampon ähnlich sehen kann. Dies ist gerade bei Verwendung des Wundpflegeartikels in Wundtaschen von Vorteil. Die verbleibende offene Seite kann vernäht, verklebt oder auch offen gelassen werden. Man kann so in einer bevorzugten Ausführungsform einen tampon- oder wurstförmigen Absorptionskörper mit innen liegenden Nähten erhalten, der sich gut eignet, um in eine Wundtasche eingeführt zu werden. Ggf. kann dieser auch mit einem Zugbändchen versehen sein, mit dessen Hilfe der Absorptionskörper nach Verwendung aus der Wundtasche herausgezogen werden kann

Grundsätzlich kann in einer anderen bevorzugten Ausführungsform der absorbierende Körper- in Draufsicht auf die Flachseite der Hülle gesehen - wesentlich kleiner als das durch die Nähte begrenzte Feld der Hülle sein. In Extremfall kann jedoch, falls in der Form einer Matte vorliegt, nahezu bis zur Naht reichen. In Hinblick auf die rasche Volumenzunahme des absorbierenden Körpers ist es günstiger, einen peripheren Abstand zwischen der Hüllennaht und den Seitenkanten der Matte zu halten, der beispielsweise im Bereich 5 cm bis 15 cm liegt.

Bevorzugt ist ferner vorgesehen, dass die Hülle asymmetrisch aufgebaut ist, dergestalt, dass der Bereich der Hülle, der in an den Körper eines Patienten angelegtem Zustand des Wundpflegeartikels vom Körper des Patienten weg weist, ein hydrophobes, wasserdichtes, und/oder wasserabweisendes Material aufweist.

Besagter Bereich der Hülle dient in dieser Ausgestaltung als Durchnässungs- oder Wäscheschutz.

Erfindungsgemäß sind weiterhin die Verwendung eines erfindungsgemäßen Wundpflegeartikels zur Behandlung von chronischen Wunden, akut blutenden Wunden, und/oder traumatisch erzeugten Wunden sowie die Verwendung e eines erfindungsgemäßen Wundpflegeartikels zur operativen bzw. postoperativen Versorgung bzw. militärmedizinischen Wundversorgung und die Verwendung eines erfindungsgemäßen Wundpflegeartikels als primäre oder sekundäre Wundauflage vorgesehen.

Ferner ist erfindungsgemäß ein Kit für die Akut-, Notfall- oder Militärmedizinische bzw. die chronische Wundversorgung, aufweisend einen erfindungsgemäßen Wundpflegeartikel gemäß einem der vorherigen Ansprüche, vorgesehen

Erfindungsgemäß ist ferner ein Verfahren zur Herstellung eines erfindungsgemäßen Wundpflegeartikels vorgesehen, aufweisend die folgenden Schritte:
a) Bereitstellen von flächigen Textilbändern aufweisend Fasern mit gelbildenden Eigenschaften, sowie Fasern mit nicht gelbildenden Eigenschaften,
b) verweben, verflechten, verwirken oder verknüpfen dieser Bänder zu einem flächigen Gebilde.

Bevorzugt ist dabei vorgesehen, dass die verwendeten Textilbänder
i) jeweils ausschließlich aus Fasern mit gelbildenden Eigenschaften bzw. aus Fasern mit nicht gelbildenden Eigenschaften bestehen, oder
ii) sowohl aus Fasern mit gelbildenden Eigenschaften als auch aus Fasern mit nicht gelbildenden Eigenschaften bestehen

Besonders bevorzugt ist dabei vorgesehen, dass das Verfahren vor Schritt a) den Schritt
a1) chemische Modifikation mindestens eines Teils der Textilbänder dergestalt, dass die enthaltenen Fasern zumindest teilweise gelbildende Eigenschaften aufweisen.

Bei besagter chemischer Modifikation handelt es sich bevorzugt um ein Verfahren zur Carboxyalkylierung, besonders bevorzugt zur Carboxymethylierung, der Cellulose oder Viskose, wie oben beschrieben. Dies Verfahren eignet sich, wie oben angedeutet, lediglich für dispergierte Cellulosefasern oder für sehr schmale Cellulosebänder.

Durch die chemische Modifikation bzw. die Carboxyalkylierung der besagten Textilbänder, und das anschließende Verweben dieser Bänder zu einem flächigen Gebilde lassen sich so erfindungsgemäß erstmals großflächige Wundpflegeartikel aufweisend chemisch modifizierte Cellulose mit Größen von 10 x 10 cm und mehr herstellen.

Da carboxyalkylierte Cellulose (insbesondere Carboxymethylcellulose) generell recht fragil ist, lassen sich Carboxyalkylcellulose-Filamente oder -Garne nur unter Schwierigkeiten nachträglich zu flächigen Gebilden verarbeiten, z.B. durch Weben oder Wirken. Es ist also nur unter Schwierigkeiten möglich, Cellulose-Filamente oder -Garne erst zu carboxymethylieren und dann daraus ein flächiges Gebilde, wie es sich für die Verwendung als großflächiger Wundpflegeartikel eignet, herzustellen.

Das oben genannte Verfahren löst dieses Problem, in dem es erlaubt, zunächst ein Textilband beispielsweise aus Cellulose herzustellen, und dieses dann in einem weiteren Schritt der chemischen Modifikation (beispielsweise einer Carboxymethylierung) zu unterziehen. Auf diese Weise kann das besagte Textilband aus der (noch) ausreichend robusten Cellulosefaser (und ggf. einer weiteren nicht gelbildenden Faser, wie z.B. Nylon)hergestellt werden, und erst anschließend wird die Cellulose in CMC umgewandelt. Die nunmehr CMC enthaltenden Textilbänder lassen sich dann zu beliebig breiten flächigen Gebilden verarbeiten.

### Varianten

Vorzugsweise besteht eine ggf. vorgesehene Hülle aus zumindest teilweise auch aus einem hydrophobem Material, beispielsweise aus Polypropylen oder aus einem hydrophob ausgerüsteten Naturmaterial, wie Baumwolle. Die hydrophoben Eigenschaften der Hülle verhindern das Verkleben mit der Wundoberfläche und tragen dazu bei, dass das Wundexsudat schneller ins Innere der Hülle gelangen kann.

Die Hülle kann auch aus einem anderen Kunststoff, insbesondere einer Polyurethan- oder Polyethylenfolie oder aus künstlicher Spinnenseiden Folie hergestellt sein.

Das Material der Hülle kann derart strukturiert sein, dass die Hülle eine raue Innenfläche und eine glatte Außenfläche aufweist. Vorzugsweise ist die raue Innenfläche der Hülle durch trichterförmige Perforationen gebildet, die sich jeweils in Richtung Innenfläche verjüngen und in eine freie Öffnungskante ("Auskragung") auslaufen. Diese raue Innenfläche wirkt den Verschiebungen des Inhaltes der Hülle entgegen, so dass auf eine Fixierung mit Klebepunkten verzichtet werden kann. Dementsprechend kann die glatte Außenfläche des Hüllenmaterials durch gewölbte, sich zwischen den Perforationen erstreckende Materialabschnitte gebildet sein. Ein solches Hüllenmaterial kann im Gegensatz zu einem beidseitig ebenen als "dreidimensional" bezeichnet werden, und ist aus z.B. aus der DE102006017194 der Anmelderin der vorliegenden Anmeldung bekannt, auf deren Offenbarungsgehalt hier vollumfänglich verwiesen wird.

Bevorzugt ist überdies vorgesehen, dass die Hülle einer erfindungsgemäßen Wundauflage zumindest abschnittsweise eine adhäsive Beschichtung aufweist, und zwar bevorzugt auf der wundabgewandten Seite, mit deren Hilfe sie - z.B. mit einem Verband - im Wundbereich fixiert werden kann.

Weiterhin kann auch vorgesehen sein, dass die Hülle einen über die eigentliche Wunde überstehenden Bereich aufweist, der mit Klebestreifen zur Fixierung versehen ist.

Überdies kann auch auf der wundabgewandten Seite der Wundauflage ein flächiger Materialabschnitt vorgesehen sein, der über die eigentliche Hülle hinausgeht und wenigstens in seinen Randbereichen eine der Haut zugewandte adhäsive Beschichtung z.B. in Form von Klebestreifen oder -flächen aufweist (sog. "Island Dressing").

Besagter flächiger Materialabschnitt kann insbesondere semiokklusive bzw. semipermeable Eigenschaften aufweisen, d.h. er kann z.B. durchlässig für Feuchtigkeit sein, nicht aber für Bakterien.

Die Permeabilität für Wasserdampf liegt dabei bevorzugt im Bereich zwischen ≥ 500 g bis ≤ 16000 g m⁻² h⁻¹.

Als Klebematerialien für die oben genannten Zwecke kommen bevorzugt physiologisch akzeptable adhäsive Agenzien in Frage, wie z.B. Hydrokolloidkleber oder medizinisch unbedenkliche Klebstoffe, wie lösungsmittelfreie, biokompatible Silikonkleber oder Polyacrylatkleber sein, die eine gute Beständigkeit gegen alle gängigen Sterilisationsverfahren aufweisen.

Der Polyurethanschaum lässt sich im Gegensatz zu anderen Kunststoffen, wie Polypropylen, Polytetrafluorethylen (Teflon) und Silikon gut kleben.

In einer besonderen Ausführungsform ist vorgesehen, dass der absorbierende Körper innerhalb der Hülle unsymmetrisch, d.h. überwiegend auf einer Seite angeordnet ist.

Ein solcher Wundpflegeartikel lässt sich bevorzugt in Wundtaschen einsetzen, in denen beengte Raumverhältnisse herrschen. Dabei verbleibt der Abschnitt der Wundauflage, in dem sich der absorbierende Körper befindet, außerhalb der Wundtasche gezeigt. Durch die Kapillarkräfte werden Exsudate, die sich in einer solchen Wundtasche befinden, effektiv aufgenommen und auch hier die Wundheilung gefördert.

Bevorzugt ist weiterhin vorgesehen, dass die Wundauflage einen lateralen Einschnitt oder eine keilförmige Aussparung aufweist, dergestalt, dass sich die Ränder des Einschnitts bzw. der Aussparung überlappend anordnen lassen.

Bevorzugt ist weiterhin vorgesehen, dass die Wundauflage einen die Wunde ausfüllenden Abschnitt aufweist. Dieser kann z.B. so ausgestaltet sein, dass er bei Kontakt mit Exsudat aufquillt und die Wunde bis zum Wundboden ausfüllt.

Auf diese Weise ist die Wundauflage dreidimensional formbar, so dass sich eine konkave Form ergibt, um ihn z.B. für die Auflage an ein Gelenk, eine Extremität oder ein gekrümmtes Körperteil anzupassen. Die überlappend angeordneten Ränder des Einschnitts bzw. der Aussparung können dabei z.B. durch Klettverschlüsse, Druckköpfe, Klebestreifen oder andere geeignete Fixierungsmittel fixiert werden.

In einer anderen bevorzugten Ausgestaltung ist vorgesehen, dass die Wundauflage dreidimensional geformt ist, dergestalt, dass er für die Auflage an ein Gelenk, eine Extremität oder ein gekrümmtes Körperteil angepasst ist.

So kann die Wundauflage z.B. eine konkave Form aufweisen, um ihn z.B. für die Auflage an die Ferse eines Fußes oder an einen Ellenbogen eines Patienten anzupassen. Der absorbierende Körper, der im Inneren der Wundauflage angeordnet ist, kann dabei herausnehmbar gestaltet sein. Besonders von Vorteil ist diese Ausgestaltung, wenn der absorbierende Körper zuvor angefeuchtet wurde bzw. durch austretendes Exsudat angefeuchtet wird, da sich hier dann aufgrund der vorliegenden Superabsorbierenden Polymere ein Gel ausbildet, das polsternd wirkt und somit die schmerzfreie Lagerung des besagten Körperteils ermöglicht. Überdies passt sich die Wundauflage durch die Anfeuchtung noch besser den anatomischen Gegebenheiten an.

Bevorzugt ist überdies vorgesehen, dass die Wundauflage zumindest teilweise in einer gerollten Form vorliegt. Hier kann vorgesehen sein, dass die ursprünglich flächige Wundauflage gerollt und ggf. in ihrer gerollten Form z.B. durch Nähen, Kleben oder Schweissen fixiert wird. Eine solche Wundauflage eignet sich insbesondere für die Verwendung als Tamponade in Wundtaschen; sie weist insbesondere eine Dochtfunktion für die aufzunehmenden Exsudate auf.

Bevorzugt ist in diesem Zusammenhang außerdem vorgesehen, dass die Wundauflage außerdem mindestens einen nutritiven, mindestens einen desinfizierenden bzw. dekontaminierenden und/oder mindestens einen Proteasen hemmend wirkenden Wirkstoff und/oder Wirkstoffkomplex aufweist.

Bei dem desinfizierend wirkenden Wirkstoff und/oder Wirkstoffkomplex kann es sich z.B. um eine Zusammensetzung aus mindestens einem Vitamin oder Vitaminderivat, einem Metallion sowie einem Detergenz handeln. Ebenso kann es sich dabei um ein BLIS (bacteriocin like inhibitory substance), um ein antimikrobielles Peptid, ein Antibiotikum, ein Silberpräparat oder um beschichtete magnetische Partikel handeln. Besonders bevorzugt sind hier quaternäre Amoniumverbindungen zu nennen.

Bei dem nutritiv wirkenden Wirkstoff und/oder Wirkstoffkomplex kann es sich um eine Zusammensetzung enthaltend mindestens die Bestandteile eines enteralen und/oder parenteralen Diätetikums handeln. Ebenso kann es sich dabei um mindestens ein Wirkelement ausgewählt aus der Gruppe enthaltend Insulin, rekombinantes Insulin, Proinsulin, einen insulinähnlichen Wachstumsfaktor (Insulin-like growth factor, IGF), ein Insulinmimetikum und/oder einen diabetikerspezifischen, nicht glucose- bzw. saccharosebasierenden Energieträger handeln.

Genauso kann es sich bei dem nutritiv wirkenden Wirkstoff und/oder Wirkstoffkomplex um ein Glykolyse-Enzym handeln, so z.B. um eine Hexokinase, die Glucose in Glucose-6-Phosphat überführt und so durch Einleitung der Glycolyse den bei Diabetikern häufig erhöhten Glucosespiegel reduziert.

Bei dem Proteasen hemmend wirkenden Wirkstoff und/oder Wirkstoffkomplex kann es sich um mindestens ein Wirkelement ausgewählt aus der Gruppe enthaltend Proteasehemmer, Superabsorbierende Polymere, Antikörper gegen Matrix -Metallo-Proteasen (MMP, insbesondere gegen MMP 2, 7 und 9), Chelatoren für zweiwertige Kationen (insbesondere für Ca²⁺), Kollagen, beschichtete magnetische Partikel, Säuren, Puffer, nicht pathogene säureproduzierende Mikroorganismen, Probiotika und/oder Symbiotika handeln. Insbesondere kann dabei vorgesehen sein, dass in der Wundauflage eine Trägersubstanz vorgesehen ist, die zweiwertige Kationen (insbesondere Ca²⁺) bindet.

Weitere Zusammenhänge und Hintergründe zu den nutritiven, einen desinfizierenden bzw. dekontaminierenden und/oder Proteasen hemmend wirkenden Wirkstoffen und/oder Wirkstoffkomplexen sind in der DE102007030931 der Anmelderin der vorliegenden Anmeldung beschrieben, auf deren Inhalt hier vollumfänglich Bezug genommen wird. In der DE102007030931 sind auch weitere nutritive, desinfizierende bzw. dekontaminierende und/oder Proteasen hemmend wirkende Wirkstoffen und/oder Wirkstoffkomplexe beschrieben, die ebenfalls als in dieser Anmeldung offenbart gelten sollen.

Weiterhin kann die Wundauflage eine Zubereitung enthaltend Phagen und/oder Bestandteile derselben enthalten. Eine solche Wundauflage ist in der DE102007054127 der Anmelderin der vorliegenden Erfindung beschrieben, auf deren Inhalt hier vollumfänglich Bezug genommen wird.

Es kann überdies vorgesehen sein, dass die Wundauflage nekrolytische und/oder fibrinolytische Enzyme enthält. Ebenso kann sie Angiogenese- oder Epidermogenesefördernde Wachstumsfaktoren enthalten (insbesondere aus der Gruppe der VEGF und EGF). Ebenso kann die Wundauflage Lockstoffe für Makrophagen enthalten, die die bei der Phagentherapie freiwerdenden Phagen und Bakterrienreste (insbesondere Endotoxine) phagocytieren.

Weiterhin ist bevorzugt vorgesehen, dass mindestens ein Abschnitt der Hüllenwand der Wundauflage ein Reservoir für mindestens einen nutritiven, einen desinfizierenden bzw. dekontaminierenden, einen Proteasen hemmend wirkenden, einen blutstillend wirkenden und/oder einen wundheilungsfördernden Wirkstoff und/oder Wirkstoffkomplex aufweist.

Besagtes Reservoir kann z.B. aus einer in die Hüllenwand eingearbeiteten Tasche bestehen. Ebenso kann das Reservoir aus einem mit besagtem Wirkstoff und/oder Wirkstoffkomplex getränkten Abschnitt der Hüllenwand bestehen, oder der Wirkstoff ist in besagten Abschnitt eingepresst.

Der Wundauflage können Substanzen zugefügt sein, die den osmotischen Druck erhöhen können. Zu den Substanzen zählen z. B. Osmodiuretika, wie Mannitol.

Mineralische Ionenaustauscher, wie Zeolithe, Bentonite oder Montmarylinite, können ebenfalls Bestandteil der Wundauflage, insbesondere seiner Matte sein. Zeolithe können u. a. Schadstoffe, wie Schwermetalle, absorbieren. Überdies entfalten sie eine blutstillende Wirkung.

Ebenso kann der Artikel auch Aktivkohle enthalten. Diese ist bevorzugt in einer dünnen Lage, beispielsweise in einer Vliesschicht, dispergiert, auf der wundabgewandten Seite angeordnet, und dient insbesondere dazu, unangenehme Gerüche aus dem Wundbereich zu absorbieren.

Weiterhin kann die Wundauflage eine Zubereitung enthaltend Phagen und/oder Bestandteile derselben enthalten. Eine solche Wundauflage ist in der DE102007054127 der Anmelderin der vorliegenden Erfindung beschrieben, auf deren Inhalt hier vollumfänglich Bezug genommen wird.

Weiterhin kann Die erfindungsgemäße Wundauflage auch in ein Wundversorgungssystem zur Wunddrainage unter Einsatz von Unterdruck eingebracht sein. Solche Systeme sind z.B. in den Schriften DE202004017052, WO2006048246 und DE202004018245 der Anmelderin der vorliegenden Erfindung offenbart, deren Offenbarungsgehalt der vorliegenden Erfindung als zugehörig betrachtet sein soll.

Aus erstgenannter ist eine Vorrichtung zur Wundbehandlung unter Einsatz von Unterdruck bekannt, aufweisend ein gasdichtes Wundabdeckungselement , das im am Körper des Patienten angelegten Zustand einen zwischen der jeweiligen Wunde und dem Wundabdeckungselement verbleibenden Raum bildet, und wenigstens eine Anschlussstelle, die mit dem Raum in Kontakt steht und über welche die im Raum befindliche Luft evakuiert werden kann, wobei das Wundabdeckungselement von wenigstens einer flächenhaften, die Wundexsudate aufnehmenden Wundauflage unterlegt ist, deren Volumen im Laufe des Absorptionsprozesses zunimmt, so dass die absorbierten Wundexsudate innerhalb der Wundauflage und damit unterhalb des Wundabdeckungselementes bis zur Entfernung der Wundauflage aus dem Körper des Patienten verbleiben, die Wundauflage wenigstens eine Lage eines mit Superabsorbentien angereicherten Textilabschnittes ist, die mit einer flüssigkeitsdurchlässigen Hülle umgeben ist, und die Lage in Draufsicht auf ihre Flachseite eine Fläche hat, die 3% bis 90% kleiner als die der Hülle ist, damit sich die Wundauflage in der Nähe seiner gesamten Füllungskapazität im Querschnitt einer Kreisform annähern kann.

Aus zweitgenannter ist ein Mehrkomponentenverband zur Wundbehandlung des menschlichen oder tierischen Körpers unter Einsatz von Unterdruck bekannt, aufweisend: ein Wundabdeckungselement zur Anbringung an Haut- und Schleimhautoberfläche, wenigstens eine Anschlussstelle, die mit dem Wundraum in Kontakt steht und über welche die im Wundraum befindlichen Stoffe evakuiert werden können, wobei dieser Superabsorbierende Polymere aufweist, wobei die absorbierten Wundexsudate an Polymere gebunden im Wundraum bis zur Entfernung aus dem Wundraum verbleiben, wobei die Polymere durch ihre Bindungskapazität wechselseitige Synergien mit den subatmosphärischen Drücken unterstützen.

Aus letztgenannter ist eine Drainagevorrichtung zur Wundbehandlung unter Einsatz von Unterdruck bekannt, aufweisend ein gasdichtes, aus folienartigem Material bestehendes Wundabdeckungselement, das im am Körper des Patienten angelegten Zustand an der Hautoberfläche um den Wundbereich herum adhäsiv befestigt ist und einen zwischen der jeweiligen Wunde und dem Wundabdeckungselement verbleibenden, abgedichteten Raum bildet, wenigstens einen Drainageschlauch, der in den Raum einsetzbar ist, über den die im Raum befindlichen Stoffe evakuiert werden können, und wenigstens eine innerhalb des Raumes angeordnete, die Wundexsudate aufsaugende Wundauflage, die wenigstens eine Lage eines mit Superabsorbentien angereicherten Textilabschnittes aufweist, die mit einer flüssigkeitsdurchlässigen Hülle umgeben ist, wobei die absorbierten Wundexsudate innerhalb der Wundauflage und damit unterhalb des Wundabdeckungselementes bis zur Entfernung der Wundauflage aus dem Körper des Patienten verbleiben, und wobei das Wundabdeckungselement eine gasdicht verschließbare Behandlungsöffnung aufweist, durch die die Wundauflage in den Raum einlegbar und aus dem Raum entnehmbar ist.

Dabei kann - im Unterschied zu den in den genannten Offenbarungen gezeigten Ausführungsformen - überdies vorgesehen sein, dass im bereich der Vakuumeinrichtung kein Absorptionskörper aufweisend Superabsorbierende Polymere vorgesehen ist. Von Bedeutung hier ist vielmehr, dass die Mikrofasern - bzw. ein Mikrofasern enthaltendes Gewirke, Abstandsgewirke, Gestricke oder Vlies - als Polsterung vorgesehen ist, die den Schalenkörper der Vorrichtung unterfüttert und so für eine dauerhafte Polsterung und Durchlässigkeit für Flüssigkeiten sorgt. Hier sind insbesondere die formstabilen, nicht quellenden Eigenschaften der Mikrofasern von Vorteil, die auch unter Anlage eines Unterdrucks von herkömmlicher Weise 80 - 250 mm Hg ausreichend hohe Rückstellkräfte aufweisen. Damit stellen die erfindungsgemäß verwendeten Mikrofasern einen hervorragenden Ersatz für die hier sonst verwendeten Schaumstoffe dar, denen es insbesondere an den besagten Rückstellkräften fehlt. Eine entsprechende erfindungsgemäße Ausgestaltung ist z.B. in Fig. 10 gezeigt.

Die erfindungsgemäße Wundauflage kann überdies eine an anatomische Gegebenheiten angepasste Form aufweisen. Hierzu kann diese z.B. in Form einer Manschette ausgebildet sein; die über den einen Arm oder ein Bein oder ein Gelenk gestülpt werden kann, oder in Form eines an die Ferse, das Ellenbogengelenk oder dergleichen angepassten Verbandes.

Die erfindungsgemäße Wundauflage kann außerdem so ausgebildet sein, dass er sich zur Umlage um eine chirurgisch angelegte Leitung eignet. Hierzu kann die Wundauflage z.B. wenigstens einen Schlitz aufweisen, der es ermöglicht, die Wundauflage am Körper eines Patienten um eine Leitung (z.B. eine Drainageleitung oder einen Katheter) umzulegen. Eine solche Wundauflage ist z.B. aus der DE202006005966 der Anmelderin der vorliegenden Erfindung bekannt, deren Inhalt vollumfänglich dem Offenbarungsgehalt der vorliegenden Beschreibung hinzugefügt werden soll. Dabei kann im distalen Bereich des Schlitzes ein Steg, ein Knopf, eine Kettelnaht, eine Schweißnaht, eine perforierte Brücke oder eine andere lösbare Verbindung ("Sollbruchstelle") vorgesehen sein, die es ermöglicht, die Wundauflage in der gewohnten Art und Weise oder in der Art einer Schlitzkompresse zu verwenden.

Hier ist besonders wichtig, dass die erfindungsgemäße Wundauflage bei Flüssigkeitsaufnahme nicht anschwillt und an Volumen zunimmt, da auf diese Weise verhindert wird, dass die umgebene Leitung verengt oder blockiert wird.

Ebenso ist in diesem Zusammenhang bevorzugt vorgesehen, dass die Wundauflage mindestens ein Agenz aufweist, das die Blutung oder die Blutungsneigung einschränken kann.

Bei besagtem Agens kann es sich um mindestens einen chemisch und/oder physiologisch wirkenden Wirkstoff bzw. Wirkstoffkomplex oder um mindestens ein physikalisch wirkendes Wirkelement handeln. Eine solcher Wundauflage ist z.B. aus der DE102007030931 der Anmelderin der vorliegenden Anmeldung bekannt.

Hierzu kann die Wundauflage beispielsweise
- als im wesentlichen flacher Materialabschnitt aufweisend Absorptionsmaterial, der aus einem aufsaugenden Vlies mit darin verteilten Superabsorbierenden Polymeren sowie mindestens einem chemisch und/oder physiologisch wirkende Wirkstoff bzw. Wirkstoffkomplex ausgebildet sein,
- als oder in Kombination mit einem Druck- oder Kompressionsverband, insbesondere als Teil einer Kompressionstherapie bei Ulcus cruris venosum
- als eine Kombination aus einer primären, nicht oder nur unwesentlich absorbierenden Wundauflage, die mindestens einen chemisch und/oder physiologisch wirkenden Wirkstoff bzw. Wirkstoffkomplex aufweist, und einer peripher von dieser primären Wundauflage angeordneten sekundären Wundauflage, die Superabsorbierende Polymere enthält, wobei ggf. zwischen beiden eine Diffusionsbarriere angeordnet ist,
- in Form eines Verbandpäckchens, aufweisend eine primäre Wundauflage mit mindestens einem chemisch und/oder physiologisch wirkenden Wirkstoff bzw. Wirkstoffkomplex sowie einem an der Wundauflage angeordneten Wickelabschnitt, der zumindest abschnittsweise Superabsorbierende Polymere aufweist, und/oder
- als Materialabschnitt mit einer Längserstreckung aufweisend Absorptionsmaterial, wobei der Materialabschnitt elastisch verformbare Eigenschaften aufweist, und wobei der Materialabschnitt Superabsorbierende Polymere sowie ggf. mindestens einen chemisch und/oder physiologisch wirkende Wirkstoff bzw. Wirkstoffkomplex aufweist

Bevorzugt handelt es sich bei dem chemisch und/oder physiologisch wirkenden Wirkstoff bzw. Wirkstoffkomplex um mindestens einen Stoff bzw. eine Zusammensetzung, die Blutstillende Eigenschaften aufweist. Diese Stoffe sind unter dem Oberbegriff "Hämostatika" bekannt.

Bevorzugt handelt es sich bei dem chemisch und/oder physiologisch wirkenden Wirkstoff bzw. Wirkstoffkomplex um mindestens einen Stoff bzw. eine Zusammensetzung, die Blutstillende Eigenschaften aufweist. Diese Stoffe sind unter dem Oberbegriff "Hämostatika" bekannt.

Bei dem physikalisch wirkenden Wirkelement handelt es sich z.B. um eine Abbindung, ein Druckpolster, einen Druckverband oder einen Kompressionsverband.

### Zeichnungen

Die Erfindung ist in einigen Beispielen anhand der Zeichnung näher erläutert.

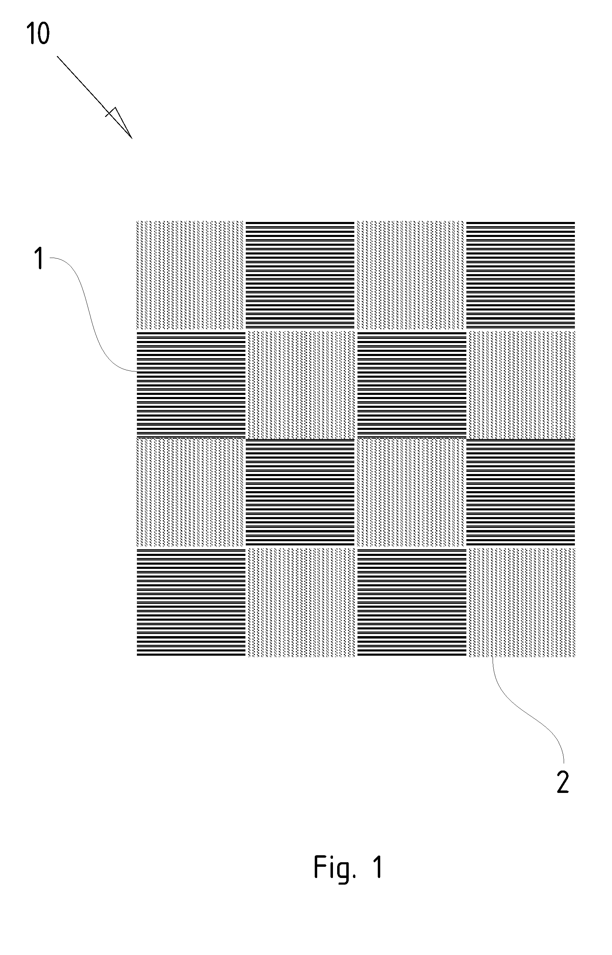

In Fig. 1 ist ein Flachgebilde 10 vergrößert dargestellt, bestehend aus rechtwinkelig miteinander verkreuzten Textilbändern 1 und 2 von einer Breite etwa 3 mm. Das Textilband 1 ist in einer an sich bekannten Webtechnik aus nicht gelbildenden Fasern, hier: aus LycraFasern, dagegen das andere Textilband 2 aus gelbildenden Carboxymethylcellulosefasern gefertigt. Das gesamte Flächengewicht des Flachgebildes 10 beträgt etwa 80 g/m².

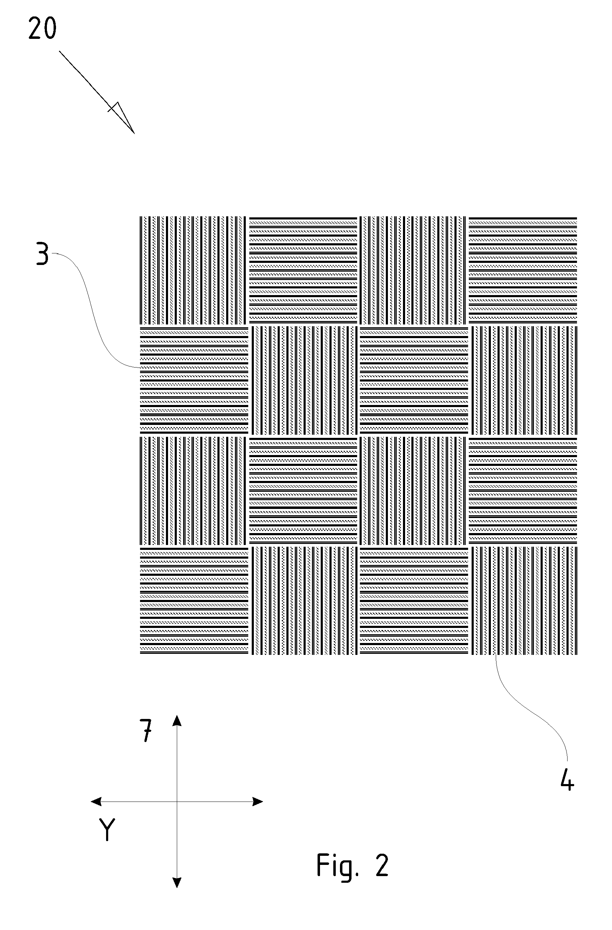

Die Fig. 2 stellt ein Flachgebilde 20 dar, bestehend aus Textilbändern 3 und 4, die ebenso rechtwinkelig verkreuzt sind, wobei die beiden Textilbänder 3, 4 jeweils aus gelbildenden (grau) und nicht-gelbildenden (schwarz) Fasern verwoben sind. Der Gewichtsanteil an nicht-gelbildenden Fasern beträgt im vorliegenden Fall 15%. Als nicht gelbildende Fasern sind Dunova-Fasern eingesetzt. Dunova ist eine synthetische Acrylfaser, die bis zu 35% Feuchtigkeit aufnehmen kann. Da die Textilbänder 3 und 4 in beiden Richtungen X, Y gleiche mechanischen Eigenschaften aufweisen, kann das Flachgebilde als homogen dehnungselastisch bezeichnet werden.

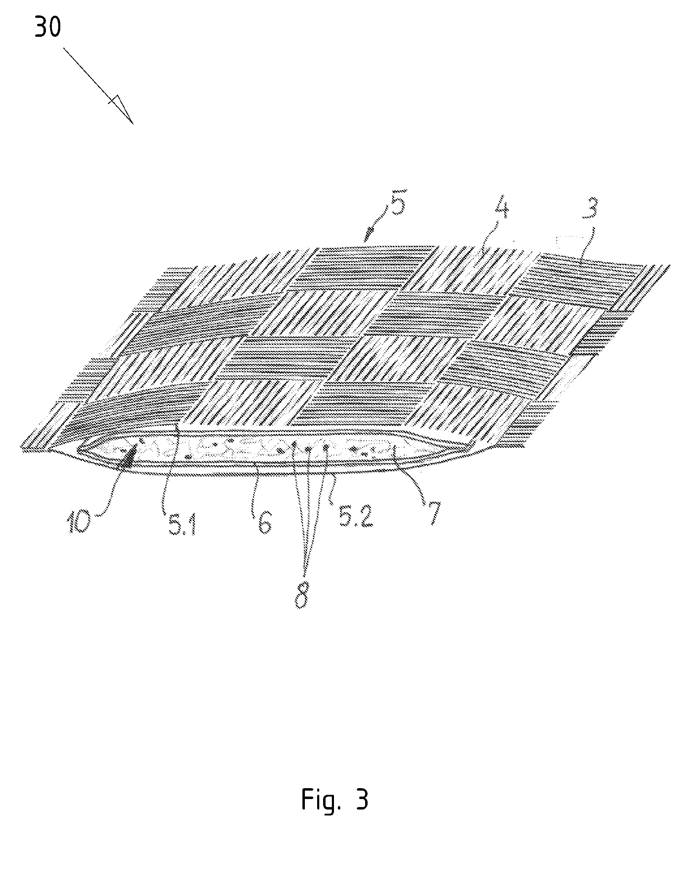

Aus den Textilbänder 3, 4 ist eine Hüllenwand 5.1 einer in Fig. 3 gezeigten Hülle 5 einer kissenförmigen Wundauflage 30 gefertigt. Die Hüllenwand 5.1 ist zur Auflage an eine nicht dargestellte Wunde gedacht, Eine der Hüllenwand 5.1 abgewandte Hüllenwand 5.2 besteht aus einer hydrophoben, jedoch atmungsaktiven Polyesterfolie. Die Hülle 5 umschließt noch einen Absorptionskörper 10, der wiederum aus einem Zellwolle-Kern 7 mit darin verteilten, pulverförmigen Superabsorberteilchen 8 und aus einer flüssigkeitsdurchlässigen Polyester-Innenhülle 6 besteht. In einem weiteren, nicht dargestellten Ausführungsbeispiel besteht der Absorptionskörper aus einer umhüllten Airlaid-Matte, die ebenfalls mit Superabsorberteilchen angereichert ist.

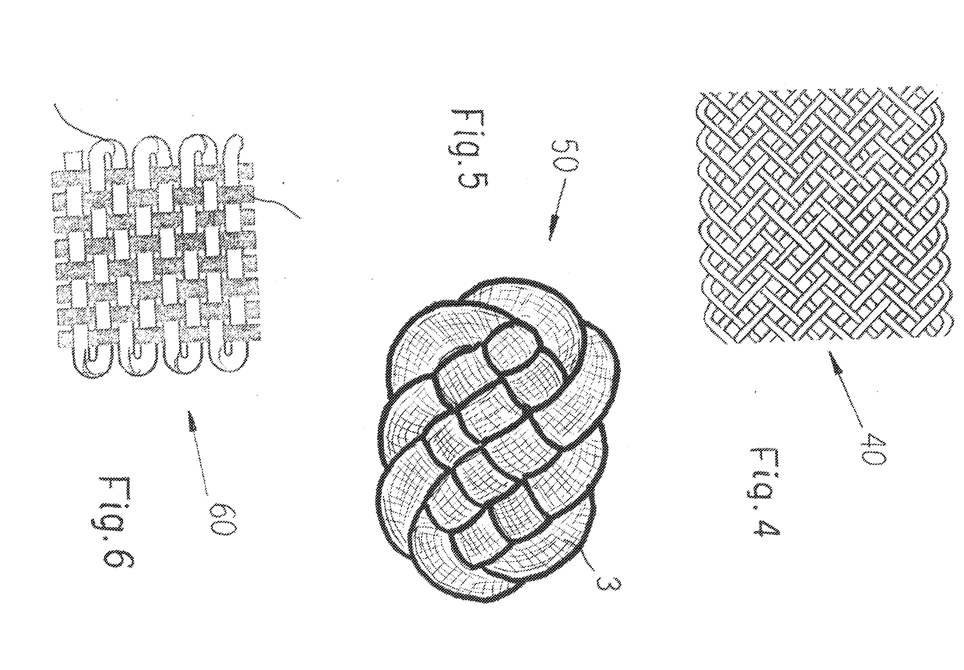

Bei der Fig. 4 handelt es sich um ein Flachgeflecht 40, bestehend aus diagonal verkreuzten Textilbändern 3, 4 gemäß Fig. 2, die Fasern mit nicht gelbildenden Eigenschaften, sowie Fasern mit gelbildenden Eigenschaften aufweisen.

Die Fig. 5 zeigt eine primäre Wundauflage 50, bestehend aus einem einzigen Textilband, das zu einer ovalen Matte zusammen verflochten ist. Das Band weist sowohl Fasern mit nicht gelbildenden Eigenschaften als auch Fasern mit gelbildenden Eigenschaften auf. Erstere verleihen dem Textilband gute mechanischen Eigenschaften, wie Reißfestigkeit, die es ermöglichen, die Wundauflage nach Quellen der gelbildenden Fasern unter Beibehaltung der Integrität der Matte aus der Wunde des Patienten problemlos abzunehmen.

Fig. 6 zeigt ein aus Textilbändern 11, 12 gefertigtes Gewebe 60, wobei die Textilbänder miteinander rechtwinkelig verkreuzt sind. Die Textilbänder 12 verlaufen in Kettrichtung. Die mäanderartig verlaufenden, aus gelbildenden Fasern zusammengesetzten Textilbänder 11 bilden eine Schussrichtung ab. Die Kettbändchen (Textilbänder 12) bestehen aus nicht gelbildenden Lycrafasern und erstrecken sich über die gesamte Bandlänge. Die Dehnbarkeit der Kettbändchen beträgt beispielsweise 160%. Die in Schussrichtung verlaufenden Textilbänder 11 weisen eine begrenzte Elastizität bzw. Dehnbarkeit auf.

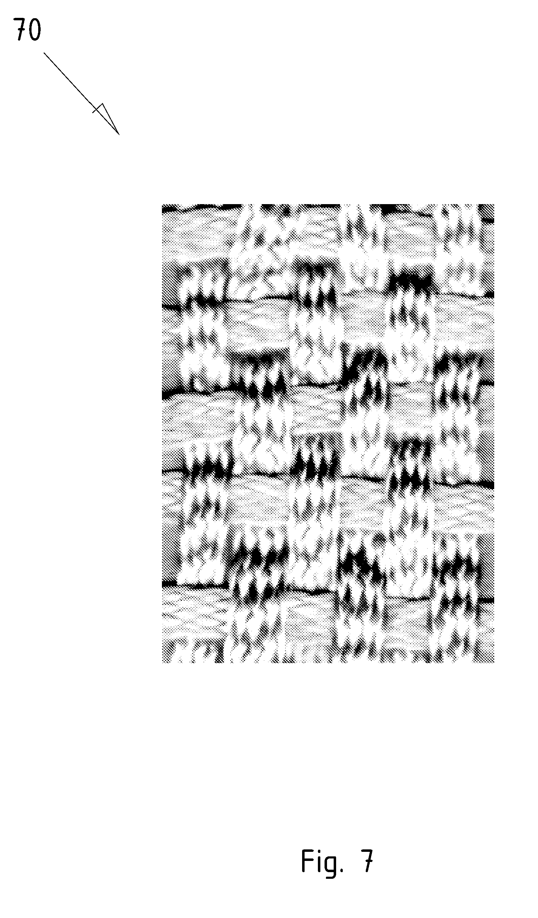

Fig. 7 zeigt eine Fotografie eines aus erfindungsgemäßen Textilbändern gefertigten flächigen Gebildes 70, das z.B. als primäre Wundauflage verwendet werden kann oder aber als Hülle für eine Wundauflage mit einem Absorptionskörper fungieren kann. Die in senkrechter Richtung verlaufenden Bänder weisen - als Fasern mit nicht gelbildenden Eigenschaften - Nylonfasern auf, während die in waagerechter Richtung verlaufenden Bändchen - als Fasern mit gelbildenden Eigenschaften - CMC-Fasern aufweisen.

Letztere Bänder können aus Cellulosefasern hergestellt worden sein, die unmodifiziert zu den Bändern verarbeitet wurden, wobei die Bänder anschließend als ganzes carboxymethyliert wurden. Alternativ können die Bänder unmittelbar aus bereits hergestellten CMC-Fasern gefertigt sein.

Es sei an dieser Stelle darauf hingewiesen, dass einzelne Textilbänder z.B. auch zu einem Zopf verflochten sein können. Aus solchen Zöpfen, die durch die partielle Schrägstellung der einzelnen Bänder eine dreidimensionale Struktur aufweisen, können dann wiederum flächige Gebilde hergestellt sein. Solche Bänder lassen sich z.B. als Wundtamponade beispielsweise in Wundtaschen oder Fistelgänge einlegen.

Ebenso können die einzelnen Bänder z.B. zu einem flächigen Gebilde verwirkt (gehäkelt, gestrickt) sein.

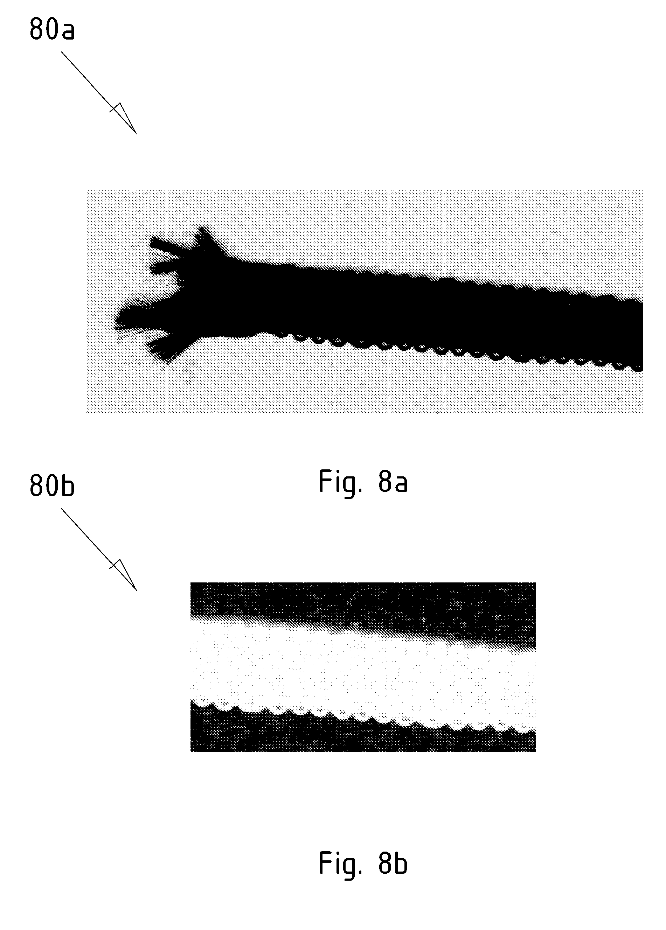

Fig. 8a zeigt eine Fotografie eines aus Nylonfasern gefertigten Textilbands 80a, das im Rahmen der vorliegenden Erfindung verwendet werden kann. Es ist im linken Bereich des Bildes gut erkennbar, dass das besagte Band nicht aus Garn hergestellt ist, sondern aus Monofilamenten.

Fig. 8b zeigt eine Fotografie eines aus CMC-Fasern gefertigten Textilbands 80b, das im Rahmen der vorliegenden Erfindung verwendet werden kann. Das Band kann aus Cellulosefasern hergestellt worden sein, die unmodifiziert zu dem Band verarbeitet wurden, wobei die Bänder anschließend als ganzes carboxymethyliert wurden. Alternativ kann das Band unmittelbar aus bereits hergestellten CMC-Fasern gefertigt sein.

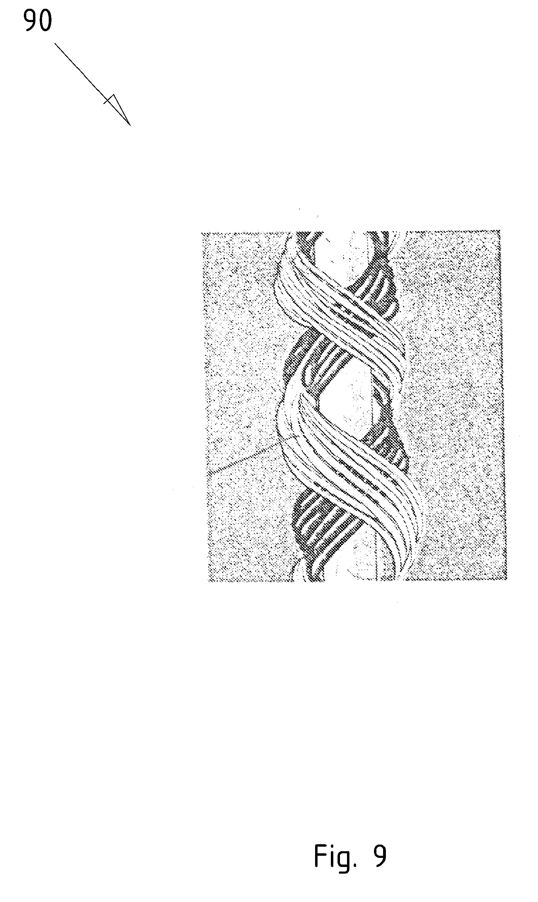

Fig. 9 zeigt eine sogenannte "Core Spun" -Ausgestaltung 90, bei welcher eine Kernfaser aus einem nicht gelbildenden Material (hier: Lycra) von gelbildenden Fasern (hier: CMC-Monofilamenten) umsponnen ist. Au dieser "Core Spun"-Faser werden dann die bereits erwähnten Textilbänder hergestellt, die wiederum für die Herstellung eines flächigen Gebildes verwendet werden.

## Patentansprüche

1. Wundpflegeartikel, aufweisend mindestens ein flächiges Gebilde enthaltend
b) Fasern mit gelbildenden Eigenschaften, sowie
c) Fasern mit nicht gelbildenden Eigenschaften,
**dadurch gekennzeichnet, dass** die Fasern in Form von flächigen Textilbändern angeordnet sind

2. Wundpflegeartikel gemäß Anspruch 1, **dadurch gekennzeichnet, dass** der Wundpflegartikel mehrere flächige Textilbänder aufweist, die miteinander verwoben, verflochten, verwirkt oder verknüpft sind.

3. Wundpflegeartikel gemäß Anspruch 1 oder 2, **dadurch gekennzeichnet, dass** die Fasern mit gelbildenden Eigenschaften auf chemisch modifizierter Cellulose basieren.

4. Wundpflegeartikel gemäß Anspruch 3, **dadurch gekennzeichnet, dass** es sich bei der chemisch modifizierten Cellulose um Carboxyalkylcellulose oder Carboxyalkylviskose handelt.

5. Wundpflegeartikel gemäß einem der vorhergehenden Ansprüche, **dadurch gekennzeichnet, dass** die Fasern mit nicht gelbildenden Eigenschaften trotz Einfluß von Feuchtigkeit ihre strukturelle Integrität aufrecht erhalten.

6. Wundpflegeartikel gemäß einem der vorherigen Ansprüche, **dadurch gekennzeichnet, dass** die Fasern mit nicht gelbildenden Eigenschaften mindestens einen Fasertyp aufweist, ausgewählt aus der Gruppe enthaltend Cellulose, Viskose, Leinen, Wolle, und/oder synthetische Fasern.

7. Wundpflegeartikel gemäß einem der vorhergehenden Ansprüche, **dadurch gekennzeichnet, dass** der Anteil an Fasern mit gelbildenden Eigenschaften in besagtem flächigen Gebilde des Wundpflegeartikels im Bereich zwischen einschließlich 50 Gew.-% und einschließlich 100 Gew.-% liegt.

8. Wundpflegeartikel gemäß einem der vorhergehenden Ansprüche, **dadurch gekennzeichnet, dass** das besagte flächiges Gebilde aufweisend flächige Textilbänder
a) eine Hülle um einen absorbierenden Körper ausbildet,
b) die Form eines Tupfers ausbildet, und/oder
c) die Form eines Wundtuchs, einer Wundauflage, einer Wundkompresse, eines Wundpolsters, einer Bandage oder eines Strumpfes ausbildet.

9. Wundpflegeartikel gemäß einem der vorhergehenden Ansprüche, **dadurch gekennzeichnet, dass** dieser Superabsorbierende Polymere aufweist.

10. Wundpflegeartikel gemäß Anspruch 9, **dadurch gekennzeichnet, dass** der absorbierende Körper mindestens ein Material aufweist, das ausgewählt ist aus der Gruppe enthaltend
a) eine Fasermatte, insbesondere aus einem Airlaid, mit eingearbeiteten Superabsorbierenden Polymeren,
b) ein Vlies, ein Nonwoven, ein Airlaid, eine Matte, ein Gewirke oder ein Gewebe aufweisend Fasern oder Garne aus Superabsorbierenden Polymeren,
c) ein absorbierendes Cellulosematerial
d) eine lose Füllung aufweisend Superabsorbierende Polymere, und/oder
e) eine Matte aus einem Weichschaumstoff.

11. Wundpflegeartikel gemäß Anspruch 8 - 10, **dadurch gekennzeichnet, dass** die Hülle asymmetrisch aufgebaut ist, dergestalt, dass der Bereich der Hülle, der in an den Körper eines Patienten angelegtem Zustand des Wundpflegeartikels vom Körper des Patienten weg weist, ein hydrophobes, wasserdichtes, und/oder wasserabweisendes Material aufweist.

12. Verwendung eines Wundpflegeartikels gemäß einem der vorherigen Ansprüche zur Behandlung von chronischen Wunden, akut blutenden Wunden, und/oder traumatisch erzeugten Wunden.

13. Verwendung eines Wundpflegeartikels gemäß einem der vorherigen Ansprüche zur operativen bzw. postoperativen Versorgung bzw. militärmedizinischen Wundversorgung.

14. Verwendung eines Wundpflegeartikels gemäß einem der vorherigen Ansprüche als primäre oder sekundäre Wundauflage.

15. Kit für die Akut-, Notfall- oder Militärmedizinische bzw. die chronische Wundversorgung, aufweisend einen Wundpflegeartikel gemäß einem der vorherigen Ansprüche.

16. Verfahren zur Herstellung eines Wundpflegeartikels gemäß einem der vorherigen Ansprüche, aufweisend die folgenden Schritte:
a) Bereitstellen von flächigen Textilbändern aufweisend Fasern mit gelbildenden Eigenschaften, sowie Fasern mit nicht gelbildenden Eigenschaften,
b) verweben, verflechten, verwirken oder verknüpfen dieser Bänder zu einem flächigen Gebilde.

17. Verfahren gemäß Anspruch 16, **dadurch gekennzeichnet, dass** die verwendeten Textilbänder
ii) jeweils ausschließlich aus Fasern mit gelbildenden Eigenschaften bzw. aus Fasern mit nicht gelbildenden Eigenschaften bestehen, oder
iii) sowohl aus Fasern mit gelbildenden Eigenschaften als auch aus Fasern mit nicht gelbildenden Eigenschaften bestehen

18. Verfahren gemäß einem der Ansprüche 16 - 17, aufweisend vor Schritt a) den Schritt a1) chemische Modifikation mindestens eines Teils der Textilbänder dergestalt, dass die darin enthaltenen Fasern zumindest teilweise gelbildende Eigenschaften aufweisen.
